# EUROPEAN PATENT SPECIFICATION

(11) **EP 1 148 779 B1**
(45) Date of publication and mention of the grant of the patent: **12.12.2007**
(21) Application number: 00903133.7
(22) Date of filing: 06.01.2000
(51) Int. Cl.: A01K 67/00, A01K 67/027, C12N 5/00, C12N 5/02, C07K 1/00, A61K 39/00

(54) **EXPRESSION OF SECRETED HUMAN ALPHA-FETOPROTEIN IN TRANSGENIC ANIMALS**
EXPRESSION VON AUSGESCHIEDENEM, MENSCHLICHEM ALPHA-FETOPROTEIN IN TRANSGENEN TIEREN
EXPRESSION D'ALPHA-FOETOPROTEINES HUMAINES SECRETEES CHEZ DES ANIMAUX TRANSGENIQUES

(30) Priority: 06.01.1999 US 114995 P
(43) Date of publication of application: 31.10.2001
(73) Proprietor: Merrimack Pharmaceuticals, Inc., Cambridge, MA 02139 (US)
(72) Inventor: LINDSAY, Stace, Cambridge, MA 02140 (US); MULROY, Robert, Cambridge, MA 02138 (US); SEMENIUK, Daniel, Lawrenceville, GA 30043 (US)
(74) Representative: Jump, Timothy John Simon
(86) International application number: PCT/US2000/000264
(87) International publication number: WO 2000/040693

(56) References cited:
- WO-A-95/00637
- WO-A-96/09377
- WO-A1-96/22787
- US-A- 5 766 884
- US-A- 5 831 141
- US-A- 5 843 776
- US-A- 6 013 857
- BENNETT ET. AL.: 'Similarity Between Natural and Recombinant Human Alpha-Fetoprotein as Inhibitors of Estrogen-Dependent Breast Cancer Growth' BREAST CANCER RESEARCH AND TREATMENT vol. 45, no. 2, September 1997, pages 169 - 179, XP002928822
- KOYAMA ET. AL.: 'Lectin Affinity Electrophoretic Demonstration of Tissue Specific and Malignant Alteration of Human a-Fetoprotein Isoform Produced in Transgenic Mice' BIOCHEMICAL AND BIOPHYSICAL RESEARCH COMMUNICATIONS vol. 223, no. 3, June 1996, pages 757 - 761, XP002928937
- DUDICH ET. AL.: 'Growth-Regulation Activity of Human Alpha-Fetoprotein for Different Types of Tumor and Normal Cells' TUMOR BIOLOGY vol. 19, no. 1, January 1998 - February 1998, pages 30 - 40, XP002928938
- DATABASE SCISEARCH 1999:701362 BIRRE ET. AL.: 'The Immunology of Alphafeprotein' & JOURNAL OF TUMOR MARKER ONCOLOGY vol. 14, no. 3, 1999, pages 55 - 62

## Description

### Background of the Invention

This invention relates to the expression and secretion of recombinant protein in transgenic animals.

Alpha-fetoprotein (AFP) is a 70 kDa glycoprotein produced by the yolk sac and fetal liver. AFP is present in fetal serum at milligram levels, and, at birth, declines to the nanogram levels normally found in adult serum: increased levels of AFP in adult serum are indicative of a yolk sac tumor, a hepatoma, or of liver regeneration. The role of AFP during fetal development is not known, although it has been suggested that AFP may protect a gestating fetus from a maternal immune attack or from the effects of maternal estrogen.

*In vitro* and *in vivo* experiments have shown that AFP has both cell growth-stimulatory and -inhibitory activities, depending upon the target cell, the relative concentration of AFP, and the presence of other cytokines and growth factors. For example, AFP can inhibit the growth of many types of tumor cells, and, in particular, inhibits estrogen-stimulated cell growth. Conversely, AFP stimulates the growth of normal embryonal fibroblasts. AFP has also been shown to have both immunosuppressive and immunoproliferative effects. In order to exploit the various biological properties of AFP, it will be necessary to obtain sufficient quantities of this molecule in an efficient and cost-effective manner.

### Summary of the Invention

In a first aspect, the invention features a substantially pure nucleic acid molecule comprising: (i) a nucleic acid sequence encoding recombinant human alpha-fetoprotein (rHuAFP), (ii) a milk-specific promoter, the promoter being operably linked to the rHuAFP-encoding sequence, and (iii) a leader sequence encoding a protein secretory signal that enables secretion of rHuAFP by milk-producing cells into the milk of a mammal.

In a second aspect, the invention features a non-human transgenic mammal that expresses recombinant human alpha-fetoprotein (rHuAFP) in its milk, wherein milk-producing cells of the mammal contain a transgene that comprises: (i) a nucleic acid sequence encoding rHuAFP, (ii) a milk-specific promoter, the promoter being operably linked to the rHuAFP-encoding sequence, and (iii) a leader sequence encoding a protein secretory signal that enables secretion of rHuAFP by milk-producing cells into the milk of a mammal.

In preferred embodiments of the second aspect of the invention, the mammal may be a goat, a cow, a sheep, or a pig.

In a third aspect, the invention features a non-human mammal's milk comprising recombinant human alpha-fetoprotein (rHuAFP). In a preferred embodiment of the third aspect of the invention, the rHuAFP is soluble and is produced by a non-human transgenic mammal whose milk-producing cells express a transgene that comprises: (i) a nucleic acid sequence encoding rHuAFP, (ii) a milk-specific promoter, the promoter being operably linked to the rHuAFP-encoding sequence, and (iii) a leader sequence encoding a protein secretory signal that enables secretion of rHuAFP by the milk-producing cells into the milk of the mammal.

In a fourth aspect, the invention features a method of producing recombinant human alpha-fetoprotein (rHuAFP) that is secreted in the milk of a mammal, comprising the steps of: (a) providing a cell transfected with a transgene that comprises: (i) a nucleic acid sequence encoding rHuAFP, (ii) a milk-specific promoter, the promoter being operably linked to the rHuAFP-encoding sequence, and (iii) a leader sequence encoding a protein secretory signal that enables secretion of rHuAFP by a milk-producing cell, wherein the milk-producing cell is derived from said transfected cell; (b) growing the cell to produce a mammal comprising milk-producing cells that express and secrete rHuAFP into milk; and (c) collecting milk containing rHuAFP from the mammal. In one preferred embodiment, the rHuAFP is purified from the milk.

In a fifth aspect, the invention features non-human mammal's milk comprising recombinant human alpha-fetoprotein (rHuAFP) for use in a method for treating a patient in need of rHuAFP.

In a preferred embodiment of the fifth aspect of the invention, the rHuAFP is produced by a non-human transgenic mammal whose milk-producing cells contain a transgene that comprises: (i) a nucleic acid sequence encoding rHuAFP, (ii) a milk-specific promoter, the promoter being operably linked to the rHuAFP-encoding sequence, and (iii) a leader sequence encoding a protein secretory signal that enables secretion of rHuAFP by milk-producing cells into the milk of the mammal.

In various preferred embodiments of the fifth aspect of the invention, the non-human mammal's milk comprising rHuAFP may be used for the treatment of cancer, for suppressing the immune system, or for inducing proliferation of bone marrow cells in a patient in need thereof.

By "human alpha-fetoprotein" or "HuAFP" or "rHuAFP" is meant a polypeptide having substantially the same amino acid sequence as the mature alpha-fetoprotein (amino acids 20-609) set forth in Genbank Accession No. J00077 and encoded by the cDNA sequence set forth in Genbank Accession No. J00077 and reported in Morinaga et al. (Proc. Natl. Acad. Sci. USA 80:4604-4608,1983).

By "human alpha-fetoprotein precursor" is meant a polypeptide having substantially the same amino acid sequence as amino acids 1-609 set forth in Genbank Accession No. J00077.

By "having substantially the same amino acid sequence" is meant a polypeptide that exhibits at least 80% identity with a naturally-occuning HuAFP amino acid sequence, typically at least about 85% identity with a naturally-occurring human HuAFP sequence, more typically at least about 90% identity, usually at least about 95% identity, and more usually at least about 97% identity with a naturally-occurring HuAFP sequence. The length of comparison sequences will generally be at least 16 amino acids, usually at least 20 amino acids, more usually at least 25 amino acids, typically at least 30 amino acids, and preferably more than 35 amino acids.

Sequence identity is typically measured using sequence analysis software with the default parameters specified therein, such as the introduction of gaps to achieve an optimal alignment (*e.g*., Sequence Analysis Software Package of the Genetics Computer Group, University of Wisconsin Biotechnology Center, 1710 University Avenue, Madison, WI 53705).

By "milk-producing cell" is meant a mammary epithelial cell that secretes milk.

By "promoter" is meant a minimal sequence sufficient to direct transcription. Also included in the invention are those promoter elements which are sufficient to render promoter-dependent gene expression controllable for cell type-specific, tissue-specific, temporal-specific, or inducible by external signals or agents; such elements may be located in the 5' or 3' or intron sequence regions of the native gene.

By "milk-specific promoter" is meant a promoter that naturally directs expression of a gene that is expressed in mammary epithelial cells, for example, the native promoter associated with the genes encoding whey acidic protein (WAP), alpha S 1-casein, alpha S2-casein, beta-casein, kappa-casein, beta-lactoglobulin, and alpha-lactalbumin.

By "recombinant HuAFP" or "rHuAFP" is meant human alpha-fetoprotein encoded by an artificially-constructed nucleic acid.

By "exogenous," as used herein in reference to a gene or a polypeptide, is meant a gene or polypeptide that is not normally present in an animal. For example, rHuAFP is exogenous to a goat.

By "purified" is meant that rHuAFP secreted into milk is partially or completely separated from other components (*e.g.,* proteins, lipids, and water) naturally found in milk, thus increasing the effective concentration of rHuAFP relative to unpurified rHuAFP found in milk.

By "substantially pure nucleic acid" is meant nucleic acid that is free of the genes which, in the naturally-occurring genome of the organism from which the nucleic acid of the invention is derived, flank the gene. The term therefore includes, for example, a recombinant DNA that is incorporated into a vector; into an autonomously replicating plasmid or virus; or into the genomic DNA of a prokaryote or eukaryote; or which exists as a separate molecule (*e.g.*, a cDNA or a genomic or cDNA fragment produced by PCR or restriction endonuclease digestion) independent of other sequences. It also includes a recombinant DNA that is part of a hybrid gene containing a nucleotide sequence not native to the gene or encoding additional polypeptide sequence, as well as the corresponding mRNA.

By "transformation" or "transfection" or "transduction" is meant any method for introducing foreign molecules into a cell. Lipofection, DEAE-dextran-mediated transfection, microinjection, protoplast fusion, calcium phosphate precipitation, transduction (*e.g.*, bacteriophage, adenoviral retroviral, or other viral delivery), electroporation, and biolistic transformation are just a few of the methods known to those skilled in the art which may be used.

By "transformed cell" or "transfected cell," or "transduced cell," is meant a cell (or a descendent of a cell) into which a DNA molecule encoding rHuAFP has been introduced, by means of recombinant DNA techniques. The DNA molecule may be stably incorporated into the host chromosome, or may be maintained episomally.

By "operably linked" is meant that a gene and one or more regulatory sequences are connected in such a way as to permit gene expression when the appropriate molecules (*e.g.*, transcriptional activator proteins) are bound to the regulatory sequences.

By "expression vector" is meant a genetically engineered plasmid or virus, derived from, for example, a bacteriophage, adenovirus, retrovirus, poxvirus, herpesvirus, or artificial chromosome, that is used to transfer an rHuAFP coding sequence, operably linked to a promoter, into a host cell, such that the encoded rHuAFP is expressed within the host cell.

By "embryonal cell" is meant a cell that is capable of being a progenitor to all the somatic and germ-line cells of an organism. Exemplary embryonal cells are embryonic stem cells (ES cells) and fertilized oocytes. Preferably, the embryonal cells of the invention are mammalian embryonal cells.

By "transgene" is meant any piece of nucleic acid that is inserted by artifice into a cell, or an ancestor thereof, and becomes part of the genome of the animal which develops from that cell. Such a transgene may include a gene which is partly or entirely exogenous (*i.e.*, foreign) to the transgenic animal, or may represent a gene having identity to an endogenous gene of the animal.

By "transgenic" is meant any cell that includes a nucleic acid sequence that has been inserted by artifice into a cell, or an ancestor thereof, and becomes part of the genome of the animal which develops from that cell. Preferably, the transgenic animals are transgenic mammals (*e.g.*, goats, sheep, cows, and pigs). Preferably the nucleic acid (transgene) is inserted by artifice into the nuclear genome (*i.e.*, a chromosome), although the transgene may also be episomally maintained (*e.g.*, carried on a vector that contains an origin of replication such as the Epstein-Barr Virus oriP).

By a "leader sequence" or a "signal sequence" is meant a nucleic acid sequence that encodes a protein secretory signal, and, when operably linked to a downstream nucleic acid molecule encoding rHuAFP, directs rHuAFP secretion. The leader sequence may be the native rHuAFP leader, an artificially-derived leader, or may obtained from the same gene as the promoter used to direct transcription of the rHuAFP coding sequence, or from another protein that is normally secreted from a cell.

By "human alpha-fetoprotein secretory signal" or "human alpha-fetoprotein signal peptide" or "human alpha-fetoprotein leader" or "human alpha-fetoprotein signal sequence" is meant a polypeptide having substantially the same amino acid sequence amino acids 1-19 set forth in Genbank Accession No. J00077. The protein secretory signal is cleaved from HuAFP during protein maturation and extracellular secretion.

By "therapeutically-effective amount" is meant an amount of recombinant human alpha-fetoprotein or fragment thereof that when administered to a patient inhibits or stimulates a biological activity modulated by human alpha-fetoprotein. Such biological activities include inhibiting the proliferation of a neoplasm or an autoreactive immune cell, or stimulating proliferation of a cell (*e.g.*, a bone marrow cell). The therapeutically-effective amount may vary depending upon a number of factors, including medical indication, the length of time of administration and the route of administration. For example, rHuAFP can be administered systemically in the range of 0.1 ng - 10g/kg body weight, preferably in the range of 1 ng - 1 g/kg body weight, more preferably in the range of 10 ng - 100mg/kg body weight, and most preferably in the range of 1 µg-10 mg/kg body weight.

### Brief Description of the Drawings

Figure 1 is a diagram showing the structure of a goat beta-casein/rHuAFP transgene for expression and secretion of rHuAFP into milk.
Figure 2 is a diagram showing the genomic organization of the human AFP gene and the two overlapping lambda (λ) fragments.
Figure 3 is a diagram showing the design of a vector including the 5' subclone of the human AFP gene and an expression construct.
Figure 4 is a diagram showing the design of a vector including the 3' subclone of the human AFP gene and an expression construct.
Figure 5 is a diagram showing a strategy for linking the 5' and 3' AFP gene fragments and inserting the entire human AFP genomic fragment into the GTC beta-casein expression vector.

### Detailed Description of the Invention

The present invention features a process for expressing secreted recombinant human alpha-fetoprotein (rHuAFP) in transgenic mammals, particularly ruminants (*e.g.,* cattle, sheep, and goats), The transgene that directs expression of secreted rHuAFP contains the human AFP coding region fused downstream of a nucleic acid containing a transcriptional promoter. Between the promoter and the protein coding region is a leader sequence encoding a protein secretory signal. Depending upon the promoter and secretory signal employed, the transgene-encoded rHuAFP is secreted into the milk of the transgenic animal. Additional nucleic acid elements, such as transcriptional enhancers, transcriptional and translational terminator sequences, 3' untranslated regions that enhance mRNA stability, and introns that enhance expression may also be included in the transgenic construct.

Production of rHuAFP by secretion into milk facilitates its purification and obviates removal of blood products and culture medium additives, some of which may be toxic, carcinogenic, or infectious. Moreover, milk containing rHuAFP may be directly consumed by humans or other mammals.

### Transgene constructs

Useful promoters for the expression of a rHuAFP transgene in mammary tissue include promoters that naturally drive the expression of mammary-specific proteins, such as milk proteins, although any promoter that permits secretion of the transgene product into milk may be used. These include, *e.g.,* the promoters that naturally direct expression of whey acidic protein (WAP), alpha S 1-casein, alpha S2-casein, beta-casein, kappa-casein, beta-lactoglobulin, and alpha-lactalbumin (see, *e.g.,* Drohan et al., U.S.P.N. 5,589,604; Meade et al. U.S. Patent No.4, 873,316; and Karatzas et al., U.S. patent No. 5,780,009).

The transgene construct preferably includes a leader sequence downstream from the promoter. The leader sequence is a nucleic acid sequence that encodes a protein secretory signal, and, when operably linked to a downstream nucleic acid molecule encoding rHuAFP, directs rHuAFP secretion. The leader sequence may be obtained from the same gene as the promoter used to direct transcription of the nucleic acid molecule encoding rHuAFP (for example, a gene that encodes a milk-specific protein). Alternatively, a leader sequence encoding the native rHuAFP protein secretory signal (amino acids 1-19 of Genbank Accession No. J00077) may be employed: nucleotides 45-101 of Genbank Accession No. J00077 encode the native HuAFP protein secretory signal. Other options include use of a leader sequence that encodes a protein secretory signal from any other protein that is normally secreted from a cell, an artificial leader sequence that encodes an artificial protein secretory signal, or a hybrid leader sequence (*e.g.*, a fusion of the goat beta-casein and HuAFP leader sequences).

In addition, the transgene construct preferably includes a transcription termination site, a signal for polyadenylation of the transcribed mRNA, and a translation termination signal. The transgene may also encode any 3' untranslated region (UTR), which increases stability of the rHuAFP mRNA, for example, a 3' UTR from the bovine growth hormone gene, a milk protein gene, or a globin gene.

The transgene construct may also include a transcriptional enhancer upstream or downstream from the transcribed region of the transgene, such as an enhancer from a viral (*e.g.*, SV40) or mammalian (*e.g.*, casein) gene.

The transgene construct may further include an intron that increases the level of expression of the transgene. The intron may be placed between the transcription initiation site and the translational start codon, 3' of the translational stop codon, or within the coding region of the transgene. The intron should include a 5' splice site (*i.e.,* a donor site), a 3' splice site (*i.e.,* an acceptor site), and preferably, at least 100 nucleotides between the two sites. Any intron that is known in the art to increase expression of a transgene (*e.g.,* an intron from a ruminant casein gene) may be used.

The transgene construct may include genomic or cDNA that expresses HuAFP or a fragment thereof. Exemplary fragments of HuAFP are described in Murgita, WO 96/2287. In addition, the transgene may be engineered to express a rHuAFP molecule that is non-glycosylated. This is accomplished by mutating the codon encoding the single N-linked glycosylation site of the AFP molecule using standard methods known in the art.

The rHuAFP transgene may be carried within a circular plasmid, a cosmid vector, or other vector, such as a vector derived from a virus. The vector may contain additional sequences that facilitate its propagation in prokaryotic and eukaryotic cells, for example, drug-selectable markers (*e.g.*, for ampicillin resistance in *E. coli,* or G-418 resistance in mammalian cells) and origins of replication (*e.g.,* colE1 for replication in prokaryotic cells, and oriP for replication in mammalian cells).

### Generation of Transgenic Animals

Transgenic constructs are usually introduced into cells by microinjection (Ogata et al., U.S. Patent No. 4,873,292). A microinjected embryo is then transferred to an appropriate female resulting in the birth of a transgenic or chimeric animal, depending upon the stage of development of the embryo when the transgene integrated. Chimeric animals can be bred to form true germline transgenic animals.

In some methods of transgenesis, transgenes are introduced into the pronuclei of fertilized oocytes. For some animals, such as mice, fertilization is performed *in vivo* and fertilized ova are surgically removed. In other animals, the ova can be removed from live, or from newly-dead (*e.g.*, slaughterhouse) animals and fertilized *in vitro.*

Alternatively, transgenes can be introduced into embryonic stem cells (ES cells). Transgenes can be introduced into such cells by electroporation, microinjection, or any other techniques used for the transfection of cells which are known to the skilled artisan. Transformed cells are combined with blastocysts from the animal from which they originate. The transformed cells colonize the embryo, and in some embryos these cells form the germline of the resulting chimeric animal (Jaenisch, R., Science 240: 1468-1474, 1988).

ES cells containing an rHuAFP transgene may also be used as a source of nuclei for transplantation into an enucleated fertilized oocyte, thus giving rise to a transgenic animal. More generally, any diploid cell derived from embryonic, fetal, or adult tissue and containing an rHuAFP transgene may be introduced into an enucleated unfertilized egg. The cloned embryo is implanted and gestated within an appropriate female, thus resulting in a fully transgenic animal (Wilmut et al., Nature 385:810-813, 1997).

In general, expression of any transgene depends upon its integration position and copy number. After a transgenic animal having the appropriate transgene expression level and tissue-specific transgene expression pattern is obtained by traditional methods (*e.g.*, pronuclear injection or generation of chimeric embryos), the animal is bred in order to obtain progeny having the same transgene expression level and pattern. There are several limitations to this approach. First, transmission of the transgene to offspring does not occur in transgenic chimeras lacking transgenic germ cells. Second, because a heterozygous transgenic founder is bred with a non-transgenic animal, only half of the progeny will be transgenic. Third, the number of transgenic progeny is further limited by the length of the gestation period and number of offspring per pregnancy. Finally, the number of useful transgenic progeny may be further limited by gender: for example, only female animals are useful for producing rHuAFP expressed in milk. In view of these limitations, nuclear transfer technology provides the advantage of allowing, within a relatively short time period, the generation of many female transgenic animals that are genetically identical.

Animals expressing rHuAFP in their milk also may be generated by direct transfer of the transgene into the mammary tissue of post-partum animals (Karatzas et al., U.S. patent No. 5,780,009). Such animals do not contain the transgene within their germline, and hence do not give rise to transgenic progeny.

### Screening for Transgenic Animals

After the candidate transgenic animals are generated, they must be screened in order to detect animals whose cells contain and express the transgene. The presence of a transgene in animal tissues is typically detected by Southern blot analysis or by employing PCR-arnplification of DNA from candidate transgenic animals (see, *e.g.*, Ausubel et al., Current Protocols in Molecular Biology, John Wiley & Sons, New York, NY, 1998; see also Lubon et al., U.S.P.N. 5,831,141). rHuAFP expression in milk may be determined by any standard immmunological assay, for example, ELISA or Western blotting analysis, using an anti-AFP antibody (see, *e.g.*, Murgita et al., U.S.P.N. 5,384,250 and Ausubel *et al., supra*). For a working example of ELISA-based detection of transgene-encoded protein in milk, see Drohan et al., U.S.P.N. 5,589,604.

### Purification of AFP from Milk

Recombinant protein may be purified from milk using standard protein purification techniques, such as affinity chromatography (see, *e.g.,* Ausubel et al., Current Protocols in Molecular Biology, John Wiley & Sons, New York, NY, 1998; see also Lubon et al., U.S.P.N. 5,831,141) or other methods known to those skilled in the art of protein purification. Once isolated, the recombinant protein can, if desired, be further purified by *e.g.,* by high performance liquid chromatography (HPLC; *e.g.,* see Fisher, Laboratory Techniques In Biochemistry And Molecular Biology, eds. Work and Burdon, Elsevier, 1980). Preferably, the purification is by at least 2-fold, more preferably, by at least 10-fold, still more preferably, by at least 100-fold, and most preferably, by at least 1000-fold.

### Use of rHuAFP Purified from Milk of Transgenic Animals

rHuAFP (Murgita et al., U.S.P.N. 5,384,250) in milk or purified from milk may be used as a diagnostic standard (*e.g.,* for detection of increased levels of AFP in adult human serum, which may indicate the presence of cancer or liver regeneration) or as a therapeutic. For example, rHuAFP produced by the methods of the invention may be administered to mammals to inhibit cancer cell growth, to induce bone marrow cell proliferation (for example, after a bone marrow transplant or after administration of a myelotoxic treatment such as chemotherapy), or as an immunosuppressive agent (for example, to treat systemic lupus erythematosus, myasthenia gravis, insulin-dependent diabetes mellitus, or to inhibit rejection of a transplanted organ).

rHuAFP in milk or purified from milk may be administered in an effective amount either alone or in combination with a pharmaceutically acceptable carrier or diluent, either alone or in combination with other therapeutic agents by any convenient means known to skilled artisans, *e.g.*, intravenously, orally, intramuscularly, or intranasally.

### Example I: Generation of Transgenic Goats Expressing Recombinant Human AFP (rHuAFP)

### Transgene construction and generation of transgenic goats

Transgenic goats expressing rHuAFP in their milk, under the control of the goat beta-casein promoter, are generated as follows. A DNA fragment containing the full length coding region of human AFP and lacking the translational start sequence is obtained by performing polymerase chain reaction (PCR) amplification using a plasmid containing the HuAFP cDNA (Genbank Accession No. J00077), such as pHuAFP (described in Murgita et al., U.S.P.N. 5,384,250) as a template and the following oligonucleotide primers: NH₂ (5'-AAA CTC GAG AAG TGG GTG GAA-3') and COOH (5-AAA CTC GAG TTA AAC TCC CAA AGC-3').

Each PCR reaction contains 34 µl DNA template, 10 µl of 10 pmol/µl 5'-primer, 10 µl 10X reaction buffer, 20 µl ImM dNTP's, 2 µl DMSO and 1 µl DNA template, 10 µl of 10 pmol/µl of 10 pmol/µl 5'primer, 10 µl of 10 pmol/µl 3'-primer, 1 µl glycerol, 10 µl DMSO and 1 µl *Pfu* DNA polymerase. Annealing, extension, and denaturation temperatures are 50°C, 72°C and 94 °C, respectively, for 30 cycles, using the Gene Amp PCR System 9600. The 1783-bp DNA obtained from the PCR reactions is digested with *Xho* I and then purified by isolating the fragment from a 0.7% TAE agarose gel, followed by gel extraction employing the Geneclean method (Bio 101; Vista, CA) according to the manufacturer's instructions.

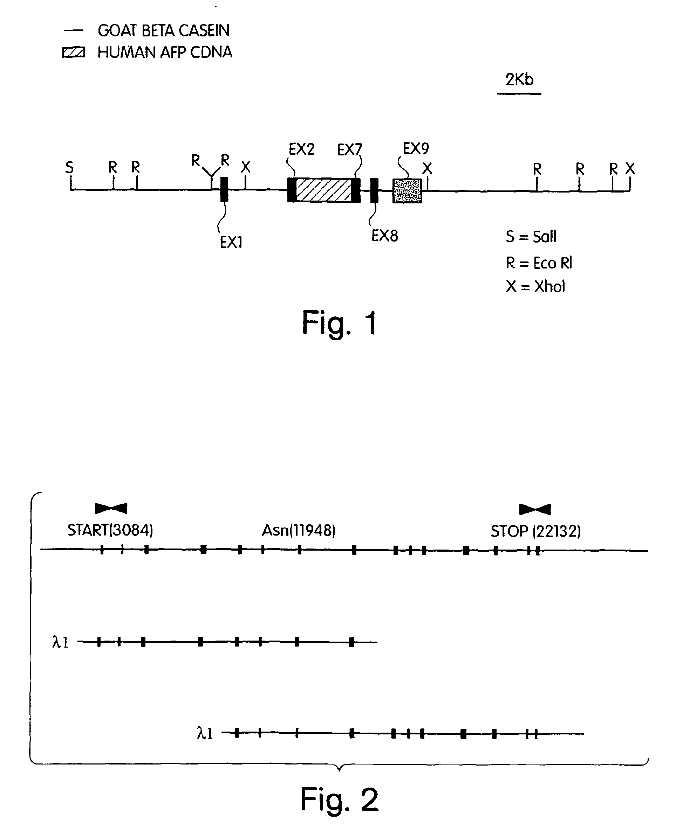

The transgene vector (see Figure 1; see Meade et al., U.S.P.N. 5,827,690) contains an altered goat beta-casein gene with an *Xho* I site in place of the coding portion of the gene. The portion deleted from the goat beta-casein gene extends from the *Taq* I site in exon 2 to the *Ppu* MI site in exon 7. Exon 2 contains the translational start codon in addition to a 15 amino acid secretion signal. To generate the goat beta-casein/human AFP transgene, the *Xho* I/*Xho* I HuAFP cDNA is ligated between exons 2 and 7 of the goat beta-casein gene at the *Xho* I site. The complete transgene contains 6.2 kb of 5' goat beta-casein sequence, the 1.8 kb HuAFP cDNA, and the 7.1 kb 3' goat beta-casein flanking sequence.

Transgenic goats are generated by injecting, into the pronucleus of collected embryos, the 15.1 kb fragment of the goat beta-casein-HuAFP purified free from procaryotic DNA at a concentration of 1.0 µg/ml in 10 mM Tris, pH 7.5, 0.1 mM EDTA. Injected embryos are then transferred to recipient females. A founder (Fₒ) transgenic goat is identified by analyzing genomic DNA from blood by polymerase chain reaction (PCR) and by Southern blot analysis in order to detect the presence of the transgene. For PCR analysis, the same two oligonucleotides that are employed to generate the HuAFP cDNA are used in the reaction. For Southern blot analysis, the DNA is fractionated on a 1% TBE agarose gel, blotted onto nitrocellulose, and probed with a random-primed ³²P-labelled 1.8 kb HuAFP cDNA. The identified founder is then bred to a nontransgenic animal to produce transgenic offspring. Alternatively, transgenic offspring may be obtained by nuclear transfer, as described above. Transmission of the transgene is detected by analyzing genomic DNA from blood as described above.

### Lactation induction

Female animals twelve months of age or older are induced to lactate by hormone therapy and hand stimulation over a 12 day period. During the first 4 days, the animal receive subcutaneous injections of 0.1 mg/kg of estradiol 17-β and 0.25 mg/kg of progesterone dissolved in 100% ethanol. This daily amount is divided between morning and evening injections. The udder is palpated once daily and the teats are hand-stimulated for 5-10 minutes each morning. Lactating transgenic females are milked manually twice per day and the milk is stored frozen at -20°C.

### Protein purification

Transgenic goat milk containing rHuAFP is thawed and the pH adjusted to 4.4 with glacial acetic acid to precipitate out the casein. The resultant precipitate is removed by centrifugation at 8000 x g for 20 min. at 4°C. The supernant is adjusted to pH 5.5 with NaOH and filtered through a 22 µm filter. The rHuAFP is purified from the whey fraction by applying the filtrate to a Butyl-Toyopearl column which is equilibrated in 0.2 M sodium phosphate, 0.1 M arginine-HCl, 0.01 % Tween 80, pH 6.0. The rHuAFP is eluted with a solution of 0.2 M sodium phosphate, 0.1 M arginine-HCl, 70% ethylene glycol. Fractions containing rHuAFP, determined by Western blot or ELISA, are pooled and dialyzed against 30 mM Tris-HCl, pH 8.0. Final purification of rHuAFP is achieved by applying the dialyzed sample onto a Mono Q column equilibrated in 20 mM Tris-HCl, pH 8.0. Bound proteins are eluted during a step gradient from 0-100% 91 M NaCl, 20 mM Tris-HCl, pH 8.0).

### Example II: Design of a Genomic Alpha Fetoprotein Transgene Expression Construct

### Human AFP Gene Cloning

The gene for human AFP spans roughly 19 kb and contains 15 exons (14 coding) separated by 14 introns. The complete sequence of the human AFP gene has been reported by Gibbs et al. (Biochemistry 26:1332-1343, 1987) and set forth in GenBank Accession No. M16610. The gene was initially cloned in two fragments of approximately 15 kb, which were then combined, to generate the expressed protein.

A human placental genomic library (Stratagene, La Jolla, CA), with an average insert size of between 9 and 23 kb, was initially screened with a series of complementary oligonucleotide probes which recognize exons at the beginning, middle, and end of the human AFP gene. The first screen did not produce any positive clones. Two larger DNA probes were then made by using the polymerase chain reaction (PCR) to amplify regions of the beginning and end of the AFP gene from human genomic DNA (arrows, Figure 2). Subsequent screening of the library with these probes produced two overlapping lambda (λ) phage clones, of approximately 15 kb, which together span the length of the human AFP gene (Figure 2).

### Construct Design

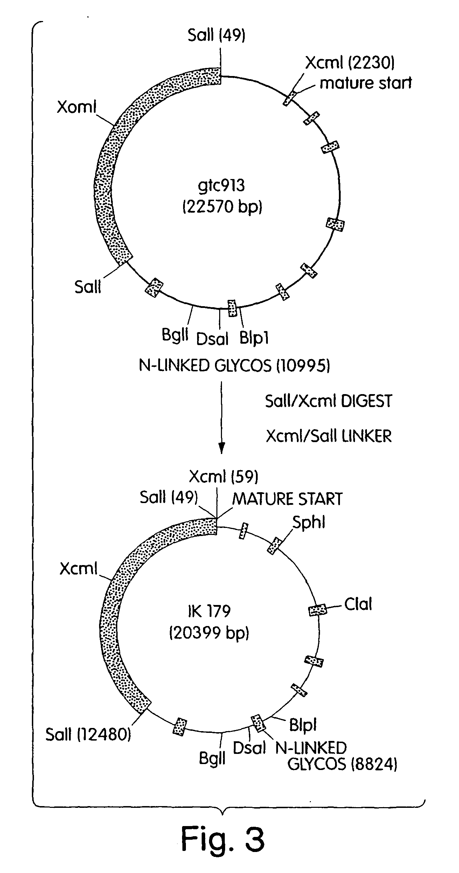

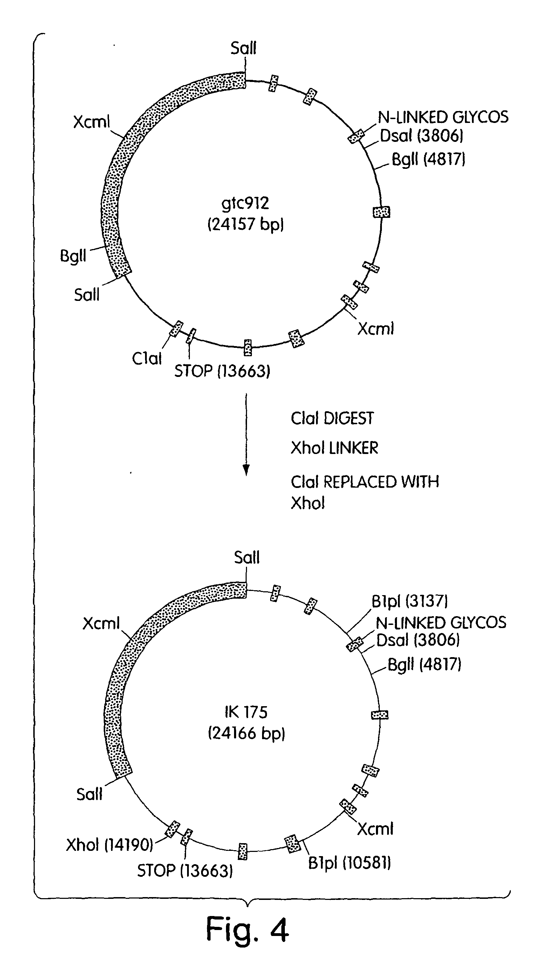

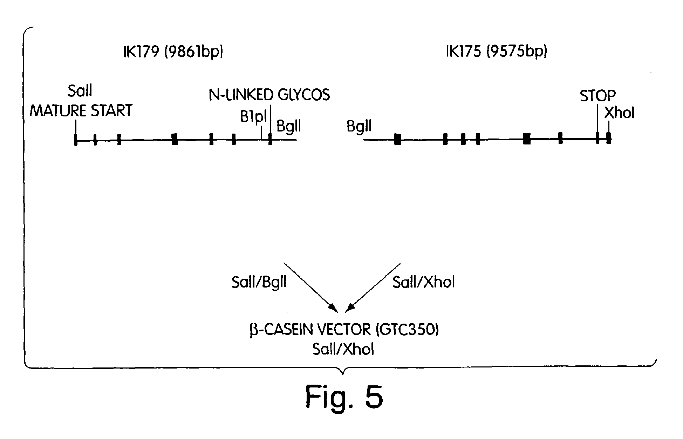

The two phage inserts were then subcloned into a superCOS I vector (this vector was used because it can accommodate larger DNA inserts). The two resulting subclones, gtc912 and gtc913 are then manipulated, as follows, to generate the final expression constructs. First, sequences 5' and 3' of the coding region are removed. In addition, at the 5' end, a Kozak sequence is added to ensure efficient initiation of translation. This is accomplished by inserting restriction enzyme "linkers" into the gene sequences for the subsequent excision of the appropriate sequences, leaving the flanking sequences intact (Figures 3&4). Second, the 5' and 3' pieces are excised from their respective vectors using an enzyme common to the two inserts which allows them to be joined together to form the complete gene. The enzyme BgII, is used since it cuts once at the 3' end of the 5' piece (IK179) and once, at the same site, at the 5' end of the 3' piece (IK175). Finally, these two pieces are linked together in a superCos plasmid vector in the SalI site and then the entire genomic fragment is placed into the SalI site of a GTC beta-casein expression vector (Figure 5).

The genomic AFP gene construct, if desired, may be mutated at its single N-linked glycosylation site. Using restriction sites flanking the glycosylation site (*e.g.*, DsaI and BlpI), an oligonucleotide containing the mutation (N to Q) can be substituted using standard molecular biological techniques (*e.g.,* gapped mutagenesis). The non-glycosylated version of the genomic AFP is then ligated into the beta-casein vector as described above and used to generate a transgenic animal, *e.g.*, a mouse, goat, sheep, pig, or cow.

The publications listed hereafter describe the generation, detection, and analysis of transgenic animals that secrete recombinant proteins into milk, as well as purification of the recombinant proteins. Hurwitz et al., U.S.P.N. 5,648,243 (goats); Meade, et al., U.S.P.N. 5,827,690 (goats); DiTullio et al., U.S.P.N. 5,843,705 (goats); Clark et al, U.S.P.N. 5,322,775 (sheep); Garner et al., U.S.P.N. 5,639,940 (sheep); Deboer et al., U.S.P.N. 5,633,076 (cows); and Drohan et al., U.S.P.N. 5,589,604 (pigs and mice).

## Claims

1. A substantially pure nucleic acid molecule comprising:
(i) a nucleic acid sequence encoding recombinant human alpha-fetoprotein (rHuAFP),
(ii) a milk-specific promoter, said promoter being operably linked to said rHuAFP-encoding sequence, and
(iii) a leader sequence encoding a protein secretory signal that enables secretion of said rHuAFP by milk-producing cells into the milk of a mammal.

2. A non-human transgenic mammal that expresses recombinant human alpha-fctoprotein (rHuAFP) in its milk, wherein milk-producing cells of said mammal contain a transgene that comprises:
(i) a nucleic acid sequence encoding rHuAFP,
(ii) a milk-specific promoter, said promoter being operably linked to said rHuAFP-encoding sequence, and
(iii) a leader sequence encoding a protein secretory signal that enables secretion of said rHuAFP by milk-producing cells into the milk of a mammal.

3. A non-human transgenic mammal as claimed in claim 2, wherein the mammal is a goar, a cow, a sheep or a pig.

4. Non-human mammal's milk comprising recombinant human alpha-feroprotein (rHuAFP).

5. Non-human mammal's milk as claimed in claim 4, wherein the rHuAFP is soluble.

6. A method of producing recombinant human alpha-fetoprotein (rHuAFP) that is secreted in the milk of a non-human mammal, said method comprising the steps of:
(a) providing a cell transfected with a transgene that comprises:
(i) a nucleic acid sequence encoding rHuAFP,
(ii) a milk-specific promoter, said promoter being operably linked to said rHuAFP-encoding sequence, and
(iii) a leader sequence encoding a protein secretory signal that enables secretion of said rHuAFP by a milk-producing cell, wherein said milk-producing cell is derived from said transfected cell,
(b) growing said cell to produce a mammal comprising milk-producing cells that express and secrete said rHuAFP into said milk, and
(c) collecting said milk containing said rHuAFP from said mammal.

7. A method as claimed in claim 6, wherein said rHuAFP is purified from said milk.

8. Non-human mammal's milk comprising recombinant human alpha-fetoprotein (rHu-AFP), for use in a method for treating a patient in need of rHuAFP.

9. Non-human mammal's milk as claimed in claim 8, wherein said non-human mammal's milk is for:
(a) the treatment of cancer,
(b) suppressing the immune system of a patient in need thereof, or
(c) inducing proliferation of bone marrow cells in a patient in need thereof.

## Patentansprüche

1. Im Wesentlichen reines Nukleinsäuremolekül, umfassend:
(i) eine Nukleinsäuresequenz, die rekombinantes humanes Alpha-Fetoprotein (rHuAFP) kodiert
(ii) einen milchspezifischen Promotor, wobei der Promotor funktionsfähig mit der rHuAFP-kodierenden Sequenz verknüpft ist, und
(iii) eine Leadersequenz, die ein Proteinsekretionssignal kodiert, das die Sekretion des rHuAFP durch milchproduzierende Zellen in die Milch eines Säugetiers ermöglicht.

2. Nicht-menschliches transgenes Säugetier, das rekombinantes humanes Alpha-Fetoprotein (rHuAFP) in seiner Milch exprimiert, wobei milchproduzierende Zellen des Säugetiers ein Transgen enthalten, das folgendes umfasst:
(i) eine Nukleinsäuresequenz, die rHuAFP kodiert
(ii) einen milchspezifischen Promotor, wobei der Promotor funktionsfähig mit der rHuAFP-kodierenden Sequenz verknüpft ist, und
(iii) eine Leadersequenz, die ein Proteinsekretionssignal kodiert, das die Sekretion des rHuAFP durch milchproduzierende Zellen in die Milch eines Säugetiers ermöglicht.

3. Nicht-menschliches transgenes Säugetier nach Anspruch 2, wobei das Säugetier eine Ziege, eine Kuh, ein Schaf oder ein Schwein ist.

4. Milch eines nicht-menschlichen Säugetiers, die rekombinantes humanes Alpha-Fetoprotein (rHuAFP) umfasst.

5. Milch eines nicht-menschlichen Säugetiers nach Anspruch 4, wobei das rHuAFP löslich ist.

6. Verfahren zur Herstellung von rekombinantem humanem Alpha-Fetoprotein (rHuAFP), das in die Milch eines nicht-menschlichen Säugetiers sekretiert wird, wobei das Verfahren die folgenden Schritte umfasst:
(a) das Bereitstellen einer Zelle, die mit einem Transgen transfiziert ist, das folgendes umfasst:
(i) eine Nukleinsäuresequenz, die rHuAFP kodiert
(ii) einen milchspezifischen Promotor, wobei der Promotor funktionsfähig mit der rHuAFP-kodierenden Sequenz verknüpft ist, und
(iii) eine Leadersequenz, die ein Proteinsekretionssignal kodiert, das die Sekretion des rHuAFP durch eine milchproduzierende Zelle ermöglicht, wobei die milchproduzierende Zelle von der transfizierten Zelle abgeleitet ist.
(b) das Anziehen der Zelle, um ein Säugetier zu erzeugen, das milchproduzierende Zellen umfasst, die das rHuAFP exprimieren und in die Milch sekretieren, und
(c) das Gewinnen der das rHuAFP enthaltenden Milch von dem Säugetier.

7. Verfahren nach Anspruch 6, wobei das rHuAFP aus der Milch aufgereinigt wird.

8. Milch eines nicht-menschlichen Säugetiers, die rekombinantes humanes Alpha-Fetoprotein (rHuAFP) umfasst, zur Verwendung in einem Verfahren zur Behandlung eines Patienten mit einem Bedarf an rHuAFP.

9. Milch eines nicht-menschlichen Säugetiers nach Anspruch 8, wobei die Milch des nicht-menschlichen Säugetiers vorgesehen ist zur:
(a) Behandlung von Krebs,
(b) Suppression des Immunsystems eines Patienten, der dessen bedarf, oder
(c) Induktion der Proliferation von Knochenmarkszellen bei einem Patienten, der dessen bedarf.

## Revendications

1. Molécule d'acide nucléique substantiellement pure comprenant :
(i) une séquence d'acide nucléique codant pour une alpha-foetoprotéine humaine recombinante (rHuAFP),
(ii) un promoteur spécifique du lait, ledit promoteur étant opérationnellement lié à ladite séquence codant pour rHuAFP, et
(iii) une séquence de tête codant pour un signal de sécrétion de protéine qui permet la sécrétion de ladite rHuAFP par des cellules productrices de lait dans le lait d'un mammifère.

2. Mammifère transgénique non humain qui exprime une alpha-foetoprotéine humaine recombinante (rHuAFP) dans son lait, dans lequel les cellules productrices de lait dudit mammifère contiennent un transgène qui comprend :
(i) une séquence d'acide nucléique codant pour rHuAFP,
(ii) un promoteur spécifique du lait, ledit promoteur étant opérationnellement lié à ladite séquence codant pour rHuAFP, et
(iii) une séquence de tête codant pour un signal de sécrétion de protéine qui permet la sécrétion de ladite rHuAFP par des cellules productrices de lait dans le lait d'un mammifère.

3. Mammifère transgénique non humain tel que revendiqué dans la revendication 2, ledit mammifère étant une chèvre, une vache, un mouton ou un porc.

4. Lait de mammifère non humain comprenant une alpha-foetoprotéine humaine recombinante (rHuAFP).

5. Lait de mammifère non humain tel que revendiqué dans la revendication 4, dans lequel la rHuAFP est soluble.

6. Procédé de production d'une alpha-foeetoprotéine humaine recombinante (rHuAFP) qui est sécrétée dans le lait d'un mammifère non humain, ledit procédé comprenant les étapes de :
(a) fourniture d'une cellule transfectée avec un transgène qui comprend :
(i) une séquence d'acide nucléique codant pour rHuAFP,
(ii) un promoteur spécifique du lait, ledit promoteur étant opérationnellement lié à ladite séquence codant pour rHuAFP, et
(iii) une séquence de tête codant pour un signal de sécrétion de protéine qui permet la sécrétion de ladite rHuAFP par une cellule productrice de lait, ladite cellule productrice de lait étant dérivée de ladite cellule transfectée,
(b) croissance de ladite cellule pour produire un mammifère comprenant des cellules productrices de lait qui expriment et sécrètent ladite rHuAFP dans ledit lait, et
(c) recueil dudit lait contenant ladite rHuAFP à partir dudit mammifère.

7. Procédé tel que revendiqué dans la revendication 6, dans lequel ladite rHuAFP est purifiée à partir dudit lait.

8. Lait de mammifère non humain comprenant une alpha-foetoprotéine humaine recombinante (rHuAFP), destiné à être utilisé dans un procédé pour traiter un patient ayant besoin de rHuAFP.

9. Lait de mammifère non humain tel que revendiqué dans la revendication 8, ledit lait de mammifère non humain étant pour :
(a) le traitement d'un cancer,
(b) la suppression du système immunitaire d'un patient en ayant besoin, ou
(c) l'induction de la prolifération de cellules de moelle osseuse chez un patient en ayant besoin.
